(19) Europäisches Patentamt
European Patent Office
Office européen des brevets

(11) Veröffentlichungsnummer: **0 352 710**
**A2**

(12) **EUROPÄISCHE PATENTANMELDUNG**

(21) Anmeldenummer: **89113622.8**

(22) Anmeldetag: **24.07.89**

(51) Int. Cl.⁴: **C07K 13/00 , C12N 15/58 , C12N 9/64 , //C12N9/72**

| | |
|---|---|
| The microorganisms have been deposited with the European Collection of Animal Cell Cultures under number 88072103 and with the Deutsche Sammlung von Microorganismen und Zellkulturen GmbH under numbers DSM 4720, 4721, 4719 and 3689.<br><br>(30) Priorität: **25.07.88 DE 3825253**<br><br>(43) Veröffentlichungstag der Anmeldung:<br>**31.01.90 Patentblatt 90/05**<br><br>(84) Benannte Vertragsstaaten:<br>**AT BE CH DE ES FR GB GR IT LI LU NL SE** | (71) Anmelder: **BOEHRINGER MANNHEIM GmbH**<br>**Sandhofer Strasse 112-132**<br>**D-6800 Mannheim Waldhof(DE)**<br><br>(72) Erfinder: **Weidle, Ulrich, Dr. rer. nat.**<br>**Landwehrstrasse 56**<br>**D-8000 München 2(DE)**<br>Erfinder: **Mattes, Ralf, Prof, Dr. rer. nat.**<br>**Friedrich-Zundel-Strasse 14**<br>**D-7000 Stuttgart 75(DE)**<br><br>(74) Vertreter: **Huber, Bernhard, Dipl.-Chem. et al**<br>**Möhlstrasse 22 Postfach 860 820**<br>**D-8000 München 86(DE)** |

(54) **t-PA-Derivat und seine Herstellung.**

(57) Ein Gewebs-Plasminogenaktivator (t-PA)-Derivat weist eine Aminosäuresequenz auf, die der des natürlichen t-PA entspricht, in der jedoch die Aminosäuren der Domäne des Kringels II fehlen. Zur Herstellung eines solchen t-PA-Derivats entfernt man aus der t-PA-DNA diejenigen Sequenzen, die für die dem Derivat im Vergleich zum kompletten t-PA-Protein fehlenden Aminosäuren kodieren, verbindet die verbleibenden DNA-Sequenzen durch Ligation, bringt sie in einen Expressionsvektor ein und exprimiert in geeigneten Wirtszellen. Ein Mittel zur Gerinnselauflösung enthält einen Gewebs-Plasminogen-Aktivator (t-PA)-Derivat, das eine Aminosäuresequenz aufweist, die der des natürlichen t-PA entspricht, in der jedoch mindestens die Aminosäuren der Domäne des Kringels II fehlen.

FIG.1

Humaner Plasminogen-Aktivator vom Gewebe-Typ

| Fibrin-finger | EGF-Domäne * | Kringel I Domäne | Kringel II Domäne | Leichte Kette (Serin-Protease) |
|---|---|---|---|---|

\* Domäne mit Homologie zu EGF (epidermaler Wachstums-Faktor)

## t-PA-Derivat und seine Herstellung

Die Erfindung betrifft neue t-PA-Derivate, Verfahren zu ihrer Herstellung, sowie Mittel zur Gerinnselauflösung, enthaltend diese t-PA-Derivate.

Geronnenes Blut enthält als Hauptkomponente der Proteinmatrix polymeres Fibrin. Dieses Fibrin wird durch Plasmin gelöst, das aus Plasminogen über Aktivierung durch die sogenannten Plasminogen-Aktivatoren gebildet wird, z. B. durch den Gewebs-Plasminogen-Aktivator t-PA (tissue-type plasminogen activator). Die enzymatische Aktivität von natürlichem oder aus Eukaryonten gentechnologisch gewonnenem t-PA (katalytische Aktivierung von Plasminogen zu Plasmin) ist in Abwesenheit von Fibrin oder Fibrin-Spaltprodukten (FSP) sehr gering, kann aber in Gegenwart dieser Stimulatoren wesentlich gesteigert werden (um mehr als den Faktor 10).

Der Wirkungsmechanismus von t-PA in vivo ist beispielsweise in Korniger und Collen, Thromb. Hämostasis 46, 561-565 (1981) beschrieben. Danach wird t-PA durch im Blut vorhandene Proteasen in eine A- und eine B-Kette gespalten und aktiviert. Dabei bleiben die beiden Teilketten über eine Cysteinbrücke verbunden. Die Stimulierbarkeit der Aktivität des t-PA ist ein entscheidender Vorteil gegenüber anderen bekannten Plasminogen-Aktivatoren, wie z. B. Urokinase oder Streptokinase (vgl. z. B. M. Hoylaerts et al., J. Biol. Chem. 257 (1982), 2912-2919; W. Nieuwenhuizen et al., Biochem. Biophys. Acta 755 (1983), 531-533).

Bei den klinischen Versuchen hat sich t-PA bereits als wirksames Mittel zur Behandlung von Thromben erwiesen, weshalb versucht wird, es in möglichst großen Mengen herzustellen.

Bei der gentechnologischen Herstellung von t-PA ist jedoch ein wesentlicher Nachteil die relativ geringe Produktionsrate eines t-PA mit der natürlichen Aminosäuresequenz in Eukaryonten und in Prokaryonten die Bildung von unlöslichen Proteinaggregaten, den sogenannten "inclusion bodies" aus denen aktives t-PA nur nach einer speziellen Reaktivierungsbehandlung gewonnen werden kann.

Der Erfindung liegt daher die Aufgabe zugrunde, t-PA-Derivate zu schaffen, welche die proteolytischen Eigenschaften und die Stimulierbarkeit der Aktivität durch Fibrin aufweisen, jedoch gegenüber einem t-PA mit der natürlichen Aminosäuresequenz bei der gentechnologischen Herstellung eine verbesserte Produktionsrate aufweisen.

Gelöst wird diese Aufgabe erfindungsgemäß durch ein t-PA-Derivat, das eine Aminosäuresequenz aufweist, die der des natürlichen t-PA entspricht, in der jedoch die Aminosäuren der Domäne des Kringel II fehlen.

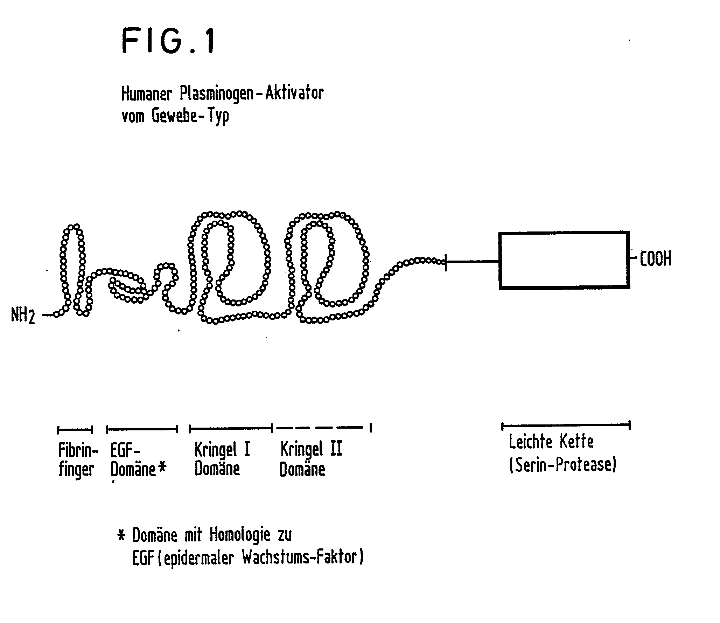

In Fig. 1 ist das natürliche t-PA mit den charakteristischen Domänen dargestellt, die vom NH₂-Ende her beginnend als Fibrinfinger-Domäne, EGF-Domäne (Epidermal Growth-Factor-like-Domäne), Kringel-Domäne I, Kringel-Domäne II, Verbindungsregion und katalytische Region bezeichnet werden.

Das erfindungsgemäße t-PA-Derivat ist nunmehr dadurch gekennzeichnet, daß die sogenannte Kringel II-Domäne fehlt. Diese Domäne ist erfindungsgemäß exakt so abgetrennt, wie es den Intron/Exon-Grenzen der natürlichen t-PA-DNA entspricht.

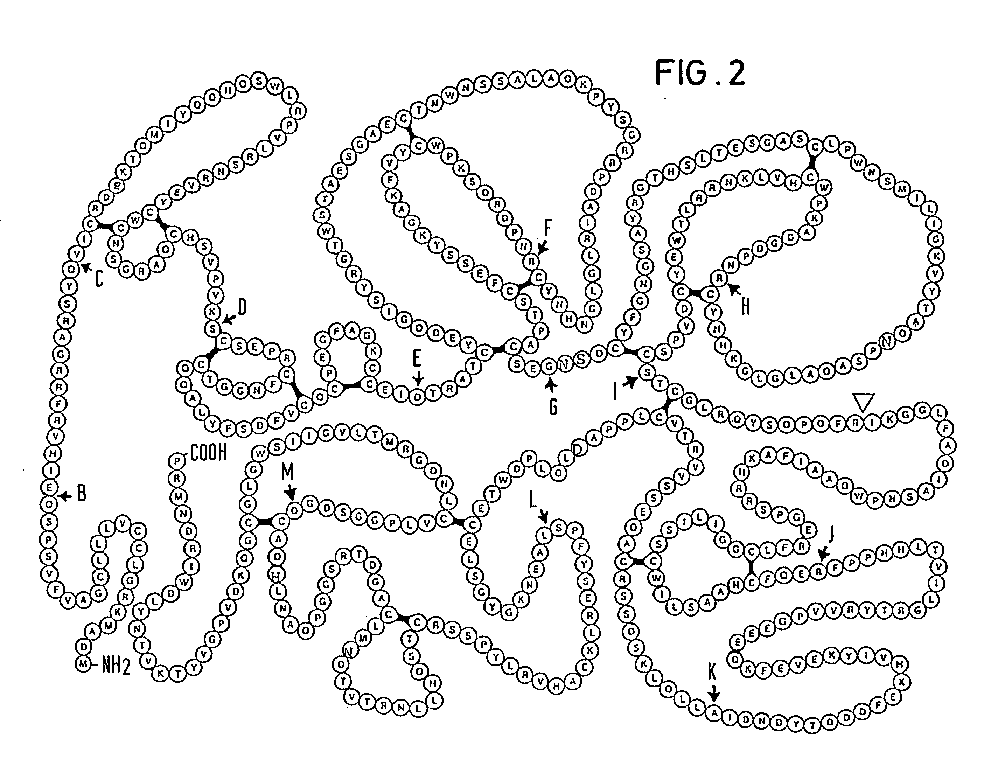

Diese Intron/Exon-Sequenzen des natürlichen t-PA sind unter Angabe der Aminosäuresequenzen und der Stellen, wo in der kodierenden DNA Introns herausgeschnitten werden (Pfeile mit Buchstaben) in Fig. 2 dargestellt, wobei am NH₂-Ende des t-PA eine aus 35 Aminosäuren bestehende Leadersequenz vorhanden ist, welche bei t-PA-Bildung in Eukaryonten in dem zuerst entstehenden t-PA-Vorläufer vorliegt.

In einer bevorzugten Ausführungsform fehlen dem erfindungsgemäßen t-PA-Derivat die Aminosäuren 176 bis 261 des natürlichen t-PA, wobei die Numerierung der Aminosäuren gemäß Pennica in Nature, 301 (1983), 214-221, erfolgt.

Das erfindungsgemäße t-PA-Derivat besitzt überraschenderweise eine wesentlich höhere Produktionsrate pro Zellmasse und Zeiteinheit in Eukaryonten, als es bei Bildung von natürlichem t-PA unter sonst gleichen Bedingungen der Fall ist. Die Stimulierbarkeit liegt zwar etwas niedriger als bei natürlichem t-PA, nämlich 5-bis 10fach im Vergleich zu 20fach, dies entspricht jedoch der Stimulierbarkeit von gentechnologisch hergestelltem t-PA der natürlichen Aminosäuresequenz.

Die Fibrin-Bindungsaktivität des gentechnologisch hergestellten t-PA-Derivats ist überraschenderweise mindestens gleich gut wie die des natürlichen t-PA.

In einer weiteren bevorzugten Ausführungsform der Erfindung fehlen dem t-PA-Derivat im Vergleich zur Aminosäuresequenz des natürlichen t-PA zusätzlich die Aminosäuren der EGF-Domäne. Bevorzugt erfolgt die Deletion der EGF-Domäne ebenfalls entsprechend den Grenzen der Intron/Exon-Prozessierungsstellen der t-PA-DNA. Dies bedeutet, daß bevorzugt in diesen t-PA-Derivaten die Aminosäuren 50 bis 87 und 176 bis 261 fehlen. Die Aminosäuren der Kringel I-Domäne, sowie die anderen Aminosäuren der Sequenz des natürlichen t-PA bleiben im erfindungsgemäßen t-PA- Derivat erhalten.

Das erfindungsgemäße t-PA-Derivat, bei dem zusätzlich die EGF-Domäne deletiert ist, besitzt zusätzlich zu dem überraschenden Effekt der erhöhten Produktionsrate in Eukaryonten eine wesentlich höhere Halbwertszeit im Organismus als das natürliche t-PA und weist weiterhin die erwähnten biologischen Eigenschaften, insbesondere die enzymatische Aktivität und Stimulierbarkeit des t-PA in unvermindertem Ausmaß auf.

Ein weiterer Gegenstand der Erfindung ist die Herstellung der erfindungsgemäßen t-PA-Derivate.

Die Herstellung der neuen t-PA-Derivate kann erfindungsgemäß dadurch erfolgen, daß man aus der t-PA-DNA diejenigen Sequenzen, die für die dem Derivat im Vergleich zum kompletten t-PA-Protein fehlenden Aminosäuren kodieren, durch Verdauung mit entsprechenden Restriktionsendonukleasen entfernt, die verbleibenden DNA-Fragmente durch Ligation verbindet, in einen Expressionsvektor einbringt und in einer geeigneten Wirtszelle exprimiert.

Zur Behandlung mit Restriktionsendonukleasen, Ligierung und Expression ist die DNA zweckmäßig in einen Vektor eingebaut. Geeignete Vektoren sind beispielsweise pBR322 und davon abgeleitete Vektoren, eukaryontische Vektoren oder Shuttle-Vektoren. Zur Wiederverknüpfung der DNA-Fragmente werden die an sich bekannten Methoden, vorzugsweise unter Verwendung von Linkern, angewendet. Die entstehenden Enden der DNA-Fragmente müssen durch geeignete Auffüllung oder durch Verdauung mit $S_1$-Nuklease oder Einführung von Linkern nämlich so modifiziert werden, daß eine phasengerechte Verknüpfung der verbleibenden Aminosäuretripletts möglich wird. Anschließend werden diese Vektoren in geeignete Wirtszellen nach an sich bekannten Verfahren eingeführt und die t-PA-Derivate exprimiert. Zwar können die erfindungsgemäßen t-PA-Derivate in Prokaryonten exprimiert werden, jedoch ist es erfindungsgemäß bevorzugt, die Expression in Eukaryonten durchzuführen, da hierin eine besonders vorteilhafte Produktionsrate erhalten wird. Hierfür wird die t-PA-DNA, falls sie nicht bereits in einem eukaryontischen Vektor vorliegt, in einen solchen vor Transformation der Wirtszellen eingebracht. Bei Expression in Prokaryonten wird als t-PA-DNA die t-PA-c-DNA verwendet, da eine korrekte Prozessierung des primären Transkripts in Prokaryonten bekanntermaßen nicht erreicht werden kann.

Erfindungsgemäß kann die Herstellung der t-PA-Derivate auch derart erfolgen, daß man t-PA-DNA in einen Vektor einbringt, den erhaltenen Vektor dann denaturiert und mit einem Oligonukleotid, welches mit den Basensequenzen, die zu beiden Seiten an die aus dem t-PA-DNA-Molekül zu entfernenden Basensequenzen anschließen, komplementäre Basensequenzen aufweist, hybridisiert, einer Reparaturmutagenese unterwirft, den so erhaltenen mutierten Vektor in eine geeignete Zelle einführt und amplifiziert, diejenigen Vektoren, in welchen die Deletion stattgefunden hat durch Hybridisierung mit einem entsprechenden Oligonukleotid ermittelt, und den Vektor in einer geeigneten Wirtszelle exprimiert.

Bevorzugt wird hierfür ein Oligonukleotid verwendet, das 18 bis 26 Nukleotide lang ist. Wiederum kann die Expression sowohl in Prokaryonten als auch in Eukaryonten ausgeführt werden, wobei für Expression in Prokaryonten als t-PA-DNA wiederum die c-DNA verwendet werden muß, oder aber die Introns der natürlichen t-PA-DNA durch zusätzliche Oligonukleotid gerichtete Mutagenese entfernt werden müssen. Bevorzugt wird jedoch die Expression in Eukaryonten durchgeführt, da wie bereits erwähnt, eine besonders günstige Produktionsrate der t-PA Muteine erreicht werden kann.

Die Selektionierung auf Vektoren, in welchen die Deletion stattgefunden hat, kann nach an sich bekannten Methoden, z. B. über Restriktionsenzymanalyse oder durch Koloniehybridisierung mit dem zur Mutagenese verwendeten Oligonukleotid, erfolgen.

In einer besonders bevorzugten Ausführungsform wird die Expression in CHO-Zellen durchgeführt.

Die Amplifikation der mutierten Vektoren findet vorzugsweise in einer Prokaryontenzelle statt, besonders bevorzugt in einem E. coli-Stamm, der ein lacI$^q$-Plasmid enthält.

Zur Expression der erfindungsgemäßen t-PA-Derivate ist es bevorzugt, daß man CHO-Zellen züchtet, die das Plasmid pSV(ΔK2)-dhfr, DSM 4721, oder das Plasmid pSV(ΔK2+EGF)-dhfr, DSM 4720, enthalten. Diese Plasmide enthalten bereits die DNA-Sequenz, welche für die erfindungsgemäßen t-PA-Derivate kodiert, nämlich ist bei pSV(ΔK2)-dhfr die für die gesamte Kringel II-Domäne kodierende Sequenz und bei pSV(ΔK2+EGF)-dhfr die gesamte Kringel-II-Domänen und EGF-Domänen kodierende Sequenz der DNA des natürlichen t-PA deletiert.

Ebenfalls Gegenstand der Erfindung sind die Plasmide p7745 und p7/4, die die für die erfindungsgemäßen t-PA-Derivate kodierenden DNA-Sequenzen enthalten und deren Herstellung in den Beispielen beschrieben ist.

Ein weiterer Gegenstand der Erfindung ist ein Mittel zur Gerinnselauflösung, enthaltend ein Gewebs-Plasminogen-Aktivator (t-PA)-Derivat, das eine Aminosäuresequenz aufweist, die der des natürlichen t-PA entspricht, in der jedoch mindestens die Aminosäuren der Domäne des Kringels II fehlen.

Bevorzugt enthält das Mittel ein t-PA-Derivat, bei dem zusätzlich die Aminosäuren der EGF-Domäne fehlen. Die Verwendung dieses t-PA-Derivats hat den besonderen Vorteil, daß aufgrund des Fehlens der EGF-Domäne die Abbaurate des Proteins im Körper geringer ist, und daher eine geringere Dosis des Proteins genügt, was vor allem deshalb vorteilhaft ist, da befürchtet wird, daß t-PA in sehr hohen Dosen als Immunogen wirken könnte. Außerdem können durch eine geringere Dosis die Behandlungskosten gesenkt werden.

Die Erfindung schafft neue, pharmakologisch wirksame Proteine, die sich vom t-PA ableiten, aber eine erhöhte Produktionsrate in Eukaryonten, verglichen mit einem in Eukaryonten exprimierten t-PA der natürlichen Aminosäuresequenz aufweist, sowie Verfahren zu ihrer Herstellung.

Die folgenden Beispiele erläutern in Verbindung mit den Abbildungen die Erfindung weiter.

Fig. 1 zeigt schematisch die Struktur des t-PA-Moleküls und die verschiedenen darauf gelegenen Domänen.

Fig. 2 zeigt die Aminosäuresequenz des natürlichen t-PA, sowie die Intron/Exon-Splicestellen.

Fig. 3 zeigt schematisch die Herstellung der Plasmide pSV($\Delta$K2)-dhfr und pSV($\Delta$K2-EGF)-dhfr.

Fig. 4 zeigt die Restriktionskarten der Plasmide p7.1, pePA 158 und pePA 159.

**Beispiel 1**

Herstellung des t-PA-Derivats $\Delta$K2 (Deletion der Kringel II-Domäne)

a) Mutagenese (site-directed mutagenesis)

Als Ausgangsplasmid diente pePA 133, (Herstellung siehe EP-A 0 242 836) das folgende Komponenten enthielt: tac-Promoter, lac-Operator, Region mit einem ATG-Start-Codon, die t-PA-Nukleotidsequenz 190-1809, den Transkriptionsterminator aus pKK223-3, ein ß-Lactamase-Gen und ein Kanamycin-Resistenz-Gen, und den Origin von pACYC 177. Zur Deletion der Kringel II-Domäne aus pePA 133 wurde die Methode von Morinaga et al., 1984, Biotechnology 2, 634, angewendet. Zunächst wurden aus pePA 133 zwei Fragmente isoliert.

Fragment A: Plasmid pePA 133 wurde mit dem Restriktionsenzym EcoRI gespalten. Die Spaltprodukte wurden gelelektrophoretisch aufgetrennt und das größte EcoRI-Fragment aus dem Gel eluiert.

Fragment B: Plasmid pePA 133 wurde mit dem Restriktionsenzym XhoI linearisiert. Das linearisierte Plasmid wurde ebenfalls via Gelelektrophorese präparativ erhalten.

Für die Mutagenese wurde folgendes Oligonukleotid synthetisch hergestellt:
5$'$ GCC TGC TCT GAG TCC ACC TGC GGC 3$'$
Zur Heteroduplex-Bildung wurden Fragment A, Fragment B (je 150 pmol) und das Oligonukleotid (75 pmol) vereinigt und in Gegenwart von 50 mmol/l NaCl, 10 mmol/l Tris, pH 7,5 und 10 mmol/l MgSO$_4$ zunächst 3 Minuten bei 100°C inkubiert und sofort auf EIS überführt. Die Renaturierung der DNA erfolgte für 30 Minuten bei 60°C. Zur Reparatursynthese wurde dem Heteroduplex-Ansatz folgendes hinzugefügt:

Desoxynukleotidtriphosphate (0,25 mmol/l), ATP (1 mmol/l), NaCl (100 mmol/l), Tris-HCl pH 7,5 (6,5 mmol/l), MgCl$_2$ (8 mmol/l), ß-Mercaptoethanol (1 mmol/l), Klenow-Fragment der DNA-Polymerase aus E. coli (0,125 U/ml Ansatz) und T4 Ligase (0,1 U/ml Ansatz). Die Reparatur-Synthese erfolgte für 4 Stunden bei 16°C. Anschließend wurde dieser Ansatz in E. coli, DSM 3689, welche ein lac I$^q$-Plasmid enthalten, transformiert. Plasmid enthaltende Transformanten wurden durch Zugabe von 25 $\mu$g/ml Kanamycin zum Nährmedium selektioniert.

Mit Hilfe der Koloniehybridisierungs-Technik unter Verwendung des oben beschriebenen Mutagenese-Oligonukleotids als Sonde konnten jene Klone ausgewählt werden, die das mutagenisierte Plasmid p7745 enthielten. Dieses Plasmid unterschied sich vom Ausgangsplasmid pePA 133 unter anderem durch das Fehlen einer EcoRI-Schnittstelle, die im Ausgangsplasmid in der Kringel II-kodierenden Region lokalisiert ist.

b) Expression des t-PA-Muteins in E. coli

Das Plasmid p 7745 wurde in E. coli Zellen, DSM 3689 transformiert, die ein lac I$^q$-Plasmid enthalten. Die Plasmid-tragenden Zellen wurden in Nährmedium bei OD 550 nm = 0,4 unter Belüftung bei 37°C kultiviert und durch Zusatz von 0,2 % Lactose oder 5 mmol/l IPTG (Isopropyl-ß-D-thiogalactopyranosid) für 3 Stunden induziert. Nach dem in EP-A-0 253 823 beschriebenen Protokoll wurden die sogenannten "inclusion bodies", das sind die unlöslichen Aggregate des hoch exprimierten t-PA-Proteins, vom Zellprotein abgetrennt und das t-PA in seine native Konfiguration zurückgeführt.

Das renaturierte Protein wurde durch SDS-Gelelektrophorese aufgetrennt und via Coomassie blue-Färbung sichtbar gemacht. Im Renaturierungsansatz war das t-PA-Mutein als dominante Bande von ca. 47 500 Dalton zu erkennen. Diese Bande zeigte - wie t-PA - Kreuzreaktivität mit Anti-t-PA-PAK aus Ziege. Das renaturierte Mutein wies sowohl amidolytische Aktivität wie auch Fibrinstimulierbarkeit auf.

c) Rekonstitution der Signalsequenz an$\Delta$K2

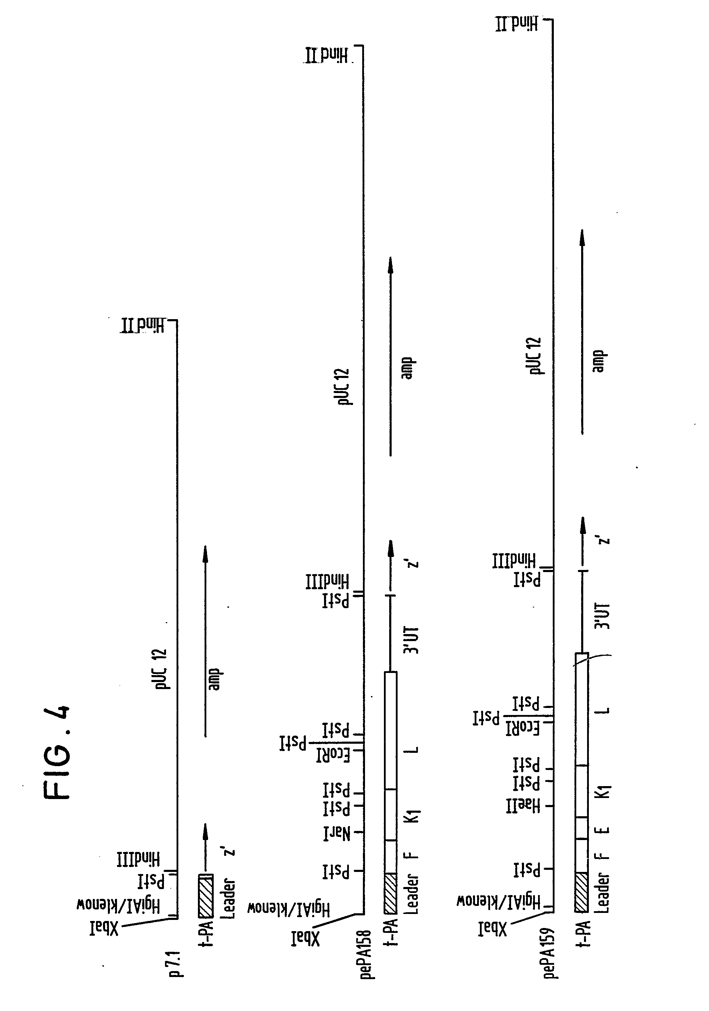

Zunächst wurde das Mutein mit der Leader-Sequenz und der 3'UT (3' untranslatierte Region), entsprechend der originalen cDNA-Sequenz, versehen. Dazu wurde Plasmid p7.1, DSM 4719, (Restriktionskarte in Fig. 4) das im Polylinker von pUC12 die 5'UT (5' untranslatierte Region) bis zu Position 77, die Leadersequenz und die N-terminale Sequenz von t-PA bis einschließlich Nukleotidposition 208 enthielt, mit PstI und Hind III gespalten (ca. 2,7 kb). Zusätzlich wurden folgende t-PA cDNA Sequenzen enthaltenden Fragmente isoliert: aus p7745 ein Pst I/Hae II-Fragment, das die Nukleotidpositionen 209 bis 421 umfaßt sowie ein Hae II/EcoRI-Fragment mit den Nukleotidpositionen 421 bis 1273, wobei durch die Mutagenese die Nukleotidpositionen 715 bis 972 deletiert sind, und aus pePA 98,1, (Herstellung siehe EP-A 0 242 836) ein Eco RI/Hind III-Fragment mit den Nukleotidpositionen 1274-2165.

Die Fragmente wurden ligiert und in E. coli, DSM 3698, transformiert. Plasmid-tragende Transformanten wurden durch Zugabe von 50 µg/ml Ampicillin im Nährmedium selektioniert. Das richtige Plasmid, mit pePA 159 bezeichnet, wurde durch Restriktionsenzym-Analyse verifiziert (Restriktionskarte in Fig. 4). Aus diesem Plasmid konnte die Δ K2 c-DNA als XbaI-HindIII-Fragment (mit Signal-Sequenz, aber ohne eigene Polyadenylierungs-Stelle) isoliert werden. Dieses Fragment enthält sieben Nukleotide 5' untranslatierte Region (5'UT) und die 3' untranslatierte Region (3'UT) bis zur BglII-Stelle bei Position 2160 (Pennica et al., Nature 301 (1983) 214-221).

**Beispiel 2**

Herstellung des t-PA-Derivats Δ(K2 + EGF)

a) Mutagenese und Expression in E. coli

Aus dem für das Mutein FEK₁L kodierenden t-PA-Vektor p 7745 wurden gemäß dem Beispiel 1 die beiden Fragmente A und B hergestellt. In diesem Fall wurde zur Heteroduplexbildung jedoch folgendes Oligonukleotid zugesetzt:
5' GTG CCT GTC AAG ACT CGG GCC ACG 3'
Dieses Oligo wurde so entworfen, daß durch die Mutagenese die Nukleotidpositionen 337 bis 450 deletiert und die Nukleotidpositionen 336, 453 und 454 dergestalt abgeändert werden, daß insgesamt eine Restriktionsschnittstelle (GANTC) entsteht. Die Nukleotid-Austausche sind so gewählt, daß kein Aminosäure-Austausch erfolgt.

Alle experimentellen Schritte wurden, wie bereits für die Konstruktion von ΔK2 in Beispiel 1 beschrieben, durchgeführt. Durch Koloniehybridisierung mit den obengenannten Mutagenese-Oligonukleotid wurden diejenigen Klone ausgewählt, die das gesuchte Plasmid pA 7/4 enthielten. Durch Restriktionsenzymanalyse, z.B. durch Nachweis der kreierten Restriktionsschnittstelle, wurde dieses Plasmid überprüft.

Das so erhaltene Muteingen Δ(K2 + EGF) konnte, wie bereits ΔK2, in E. coli exprimiert und als Protein mit einer Größe von etwa 44.000 Dalton nachgewiesen werden. Dies entspricht der reduzierten Größe des natürlichen t-PA-Moleküls, in welchem die 38 Aminosäuren der EGF-Domäne und die 86 Aminosäuren der Kringel-II Domäne deletiert sind. Das Mutein zeigte Kreuzreaktivität mit anti-t-PA-PAK aus Ziege, amidolytische Aktivität und Fibrinstimulierbarkeit.

b) Rekonstitution der Signalsequenz an Δ-(K2 + EGF)

Zur Anfügung der originalen Leader-Sequenz und der 3'UT an das Muteingen FK₁L wurden folgende Fragmente isoliert und ligiert: ein 2,7 kb großes PstI/Hind III-Fragment aus p 7,1, DSM 4719, ein ca. 0,9 bp großes HindIII/EcoRII-Fragment aus pePA 98,1 (Herstellung siehe EP-A 0 242 836), ein EcoRI/NarI-Fragment aus p 7745 mit etwa 500 bp und ein etwa 185 bp umfassendes NarI/PstI-Fragment aus pA 7/4. Der Ligationsansatz wurde in E. coli-Zellen, DSM 3689, transformiert und die Plasmid enthaltenden Transformanten durch Zugabe von 50 µg/ml Ampicillin zum Nährmedium selektioniert. Das gewünschte Plasmid pePA 158 (Restriktionskarte in Fig. 4) wurde durch detaillierte Restriktionsenzym-Analyse gefunden. Die Δ-(K2 + EGF) c-DNA konnte aus diesem Plasmid als XbaI/Hind III-Fragment isoliert werden. Dieses Fragment enthält sieben Nukleotide 5' untranslatierte Region und die 3' untranslatierte Region bis zur BglII-Stelle bei Position 2160.

**Beispiel 3**

a) Konstruktion eukaryontischer Expressionsvektoren für ΔK2 und Δ(K2 + EGF)

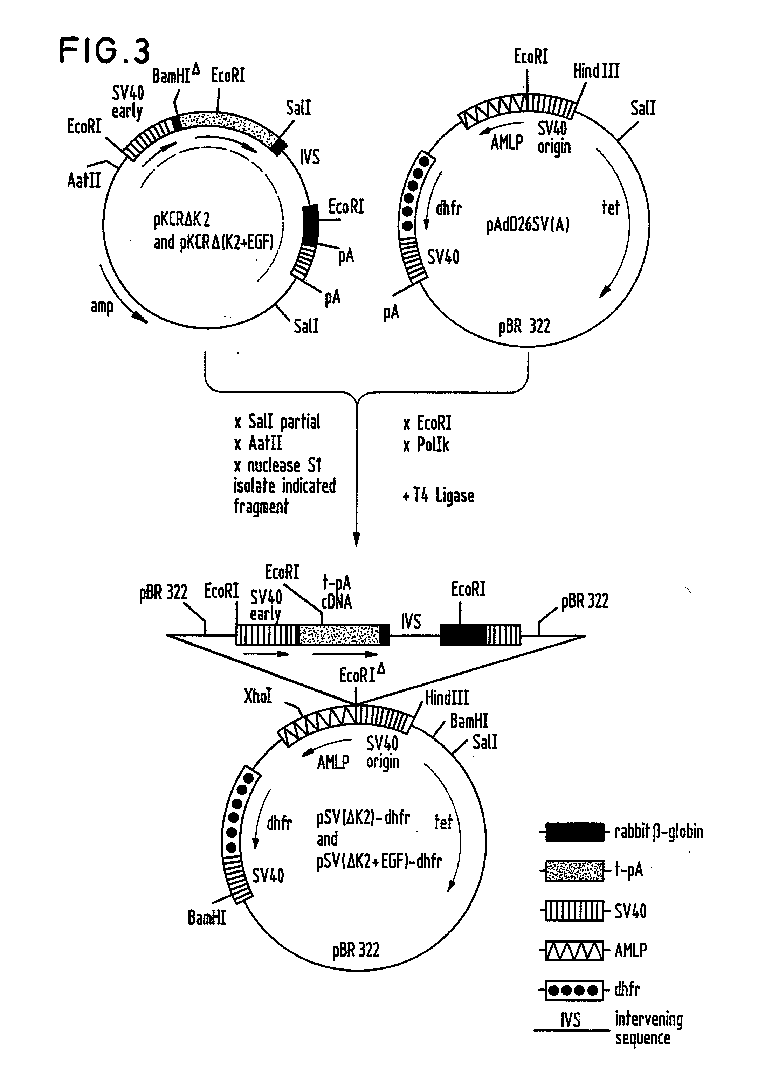

Menschliche ΔK2 und Δ(K2 + EGF) c-DNA (Pennica et al., Nature 301 (1983) 214-221) wurde in die einzige BamHI-Schnittstelle des Plasmids pKCR (O'Hare et al., Proc. Natl. Acad. Sci. USA 78 (1981), 1527-1531) als XbaI-HindIII-Fragmente insertiert, woraus sich die Plasmide pKCR(ΔK2) und pKCR(ΔK2 + EGF) ergaben. Die Enden der Fragmente wurden hierfür mit Polymerase I Klenow-Fragment aufgefüllt. Beide c-DNA'S enthielten sieben authentische Nukleotide am 5′-Ende und es fehlten ihre eigenen Polyadenylierungsstellen. Beide Plasmiden verliehen Bakterien Resistenz gegen das Antibiotikum Ampicillin (Amp). Die Expression beider c-DNA'S wird durch den SV40 early-Promotor getrieben. Auf die c-DNA folgt im Plasmid eine lange intervenierende Sequenz von Kaninchen-ß-Globin und die Polyadenilierungsstellen, die von Kaninchen-ß-Globin und SV40 stammen. Um diese Expressionskassette zu isolieren, wurden pKCR-(ΔK2) und pKCRΔ(K2 + EGF) durch partielle Spaltung mit SalI linearisiert und dann mit AatII geschnitten und die überhängenden Enden mit Nuklease S1 abgebaut. Das genannte Fragment wurde aus einem niedrigschmelzenden Agarosegel isoliert und in die aufgefüllte einzige EcoRI-Schnittstelle von pAdD26SV(A) (Kaufman and Sharp, J. Mol. Biol. 159 (1982) 601-621) einligiert. pAdD26SV(A) enthält eine Expressionskassette für Maus-DHFR-c-DNA, die durch den major late-promotor des Adenovirus 2 (AMLP) getrieben wird, den SV40-Replikationsursprung und verleiht Bakterien Resistenz gegen das Antibiotikum Tetracyclin. Die Orientierung der Expressionskassette für t-PA in den resultierenden Plasmiden pSV(ΔK2)-dhfr, DSM 4721, und pSVΔ(K2 + EGF)-dhfr, DSM 4720, wurde durch Restriktionsanalyse überprüft. Fig. 3 zeigt schematisch die Herstellung der Plasmide, sowie die Lage der einzelnen Elemente auf den Plasmiden.

b) Herstellung von Zellinien, die konstitutiv K2 und Δ(K2 + EGF) sezernieren.

CHO dhfr⁻-Zellen, ECACC 88072103, wurden wie von Urlaub und Chasin, Proc. Natl. Acad. Sci. USA 77 (1980), 4216-4220 beschrieben, gezüchtet und vermehrt. Zur DNA-Transfektion wurden Calciumphosphat-Präzipitate mit 20 μg pSV Δ(K₂)-dhfr und pSVΔ(K2 + EGF)-dhfr, wie von Graham and Van der Eb, Virology 52 (1973), 456-467 beschrieben, in einem Volumen von 4 ml hergestellt. 1 ml des Präzipitats wurde zu $3 \times 10^5$ bis $1 \times 10^6$ Zellen in 10 ml Medium in 9 cm Kulturschalen zugegeben. Die Zellen wurden 8 bis 16 Stunden mit dem Präzipitat inkubiert, dann das Medium entfernt, die Zellen mit 10 ml TBS (25 mmol Tris-HCl, pH 7,4, 137 mmol NaCl, 5 mmol KCl, 0,6 mmol $Na_2HPO_4$) gewaschen und dann im geeigneten Medium inkubiert. CHO-dhfr⁻-Zellen wurden 48 Stunden nach Transfektion 1:10 umgesetzt und dann in einem Selektionsmedium gezüchtet (Kaufman and Sharp, loc. cit.). Die Klone wurden 2 bis 3 Wochen nach der Transfektion trypsiniert, zur Massenkultur hochgezogen und die Überstände auf t-PA-Immunreaktivität durch ELISA, wie beschrieben von Weidle und Buckel in Gene 51 (1987) 31-41, untersucht. Positive Klone wurden dann gepoolt und 4 Platten mit jeweils $1 \times 10^6$ Zellen wurden in ein Medium mit 20 nmol/l Methotrexat ausgewählt. Nach 2 Wochen erschienen Methotrexat-resistente Kolonien. Sie wurden zur Konfluenz gezüchtet und 100 nmol/l Methotrexat im Medium ausgesetzt. Resistente Zellen wurden einer schrittweisen höher werdenden Methotrexat-Konzentration (300 nmol/l, 500 nmol/l, 1 μmol/l und 5 μmol/l) ausgesetzt. Klone wurden schließlich durch limitierte Verdünnung isoliert und die besten t-PA-Produzenten durch Untersuchung der Überstände auf t-PA-Immunoreaktivität durch ELISA untersucht.

c) Untersuchung von ΔK₂ und Δ(K2 + EGF) auf Fibrinstimulierbarkeit der proteolytischen Aktivität.

Die Plasminogen-Aktivator-Aktivität wurde durch eine indirekte spektrophotometrische Untersuchung (Verheijen et al., Thromb. Haemostasis 48 (1982), 266-269) untersucht. t-PA überführt Plasminogen in die aktive Serin-Protease Plasmin, die eine Bindung in einem chromogenen Substrat hydrolysiert, dessen Absorption bei 405 nm in der Folge über einen Zeitraum von bis zu 2 Stunden verfolgt wurde. Als chromogenes Substrat wurde Chromozym® PL (Tosylglycyl-prolyl-lysin-4-nitranilid-acetat) verwendet. Die Untersuchungen wurden bei 25°C in 0,1 mol/l Tris-HCl, pH 7,5, 0,15 mol/l Tween 80, enthaltend 0,3 μmol/l Plasminogen, BrCN verdautes Fibrinogen (120 μg/ml) und 0,30 mmol/l Chromozym® PL durchgeführt. Die Überstände von CHO-Zellen, die mit t-PA Expressionskonstrukten transfiziert worden waren, wiesen eine 20- bis 25fache Stimulierung der fibrinstimulierten Plasminaktivität auf. Die Überstände von CHO-Zellen, die ΔK2-konstitutiv sezernierten, wiesen eine 5- bis 10fache Stimulierung der Protease-Aktivität nach Stimulierung mit Fibrin auf. Die Überstände von CHO-Zellen, welche Δ(K2 + EGF) konstitutiv sezernierten, konnten bezüglich Plasmin-Aktivität ebenfalls 5- bis 10fach durch Fibrin stimuliert werden.

d) Produzierbarkeit von ΔK2 und Δ(K2 + EGF) in Säugerzellen (CHO)

Die Produktionsleistung von CHO-Zellen, welche t-PA (die Herstellung erfolgte, indem anstelle der verkürzten DNA die vollständige t-PA cDNA nach Pennica et al., gemäß Beispiel 3a in den Vektor eingesetzt wurde), ΔK2 (Beispiel 3b) und Δ-(K2 + EGF) (Beispiel 3b) konstitutiv sezernieren, wurde durch Aussaat von jeweils $3 \times 10^5$-Zellen (im Duplikat) und Bestimmung der t-PA-Menge nach 24 Stunden durch ELISA durchgeführt. Mikrotiterplatten der Firma Nunc mit 96 Vertiefungen wurden mit Anti-t-PA-IgG (8 μg/ml) in 50 mmol/l Kaliumphosphatpuffer, pH 7,5, beschichtet und dreimal mit PBS (0,15 mol/l NaCl, 10 mmol/l Na-phosphat pH 7,5), 0,5 % Rinderserumalbumin, gewaschen. Die Vertiefungen wurden mit 200 μl Gewebekultur-überstand 12 Stunden bei 4° C inkubiert. Die Überstände wurden sorgfältig entfernt und die Vertiefungen dreimal mit PBS gewaschen. Die Vertiefungen wurden dann mit Peroxidase-konjugiertem Ziegen-Anti-t-PA-IgG, Wasserstoffperoxid und 2,2'-Azino-di(3-ethylbenzthiazolin-sulfonat (6)) (ABTS®) inkubiert. Die Absorption bei 405 nm wurde gemessen und die Menge durch Vergleich der Absorption mit einer Eichkurve bestimmt.

Für ΔK2 ergab sich, daß die Produktion pro Zellmasse und Zeiteinheit deutlich größer ist (Faktor 3) als bei der Bildung von natürlichem t-PA unter sonst gleichen Bedingungen.

e) Messung der Fibrin-Bindungs-Affinität

t-PA, ΔK2 und Δ(K2 + EGF) aus CHO-Zellen, die diese Proteine konstitutiv sezernieren, wurden im Fibrin-Bindungstest (J. Biol. Chem. 257 (1982) 2920-2925) untersucht. Es zeigte sich, daß die Fibrin-Bindungsaktivität von ΔK2 und Δ(K2 + EGF) mindestens so gut ist wie die von natürlichem t-PA.

Ansprüche

1. Gewebs-Plasminogenaktivator (t-PA)-Derivat, **dadurch gekennzeichnet,**
daß es eine Aminosäuresequenz aufweist, die der des natürlichen t-PA entspricht, in der jedoch die Aminosäuren der Domäne des Kringels II fehlen.

2. t-PA-Derivat nach Anspruch 1, **dadurch gekennzeichnet,** daß die Kringel II-Domäne entsprechend den Intron/Exon-Grenzen der t-PA-DNA deletiert ist.

3. t-PA-Derivat nach Anspruch 1 oder 2, **dadurch gekennzeichnet,**
daß die Aminosäuren 176 bis 261 fehlen.

4. t-PA-Derivat nach einem der Ansprüche 1 bis 3, **dadurch gekennzeichnet,**
daß zusätzlich die Aminosäuren der EGF-Domäne fehlen.

5. t-PA-Derivat nach Anspruch 4, **dadurch gekennzeichnet,** daß die EGF-Domäne entsprechend den Intron/Exon-Grenzen der t-PA-DNA deletiert ist.

6. t-PA-Derivat nach Anspruch 4 oder 5, **dadurch gekennzeichnet,**
daß die Aminosäuren 50 bis 87 und 176 bis 261 fehlen.

7. Verfahren zur Herstellung eines t-PA-Derivats nach einem der Ansprüche 1 bis 6, **dadurch gekennzeichnet,** daß man aus der t-PA-DNA diejenigen Sequenzen, die für die dem Derivat im Vergleich zum kompletten t-PA-Protein fehlenden Aminosäuren kodieren, entfernt, die verbleibenden DNA-Sequenzen durch Ligation verbindet, in einen Expressionsvektor einbringt und in geeigneten Wirtszellen exprimiert.

8. Verfahren zur Herstellung eines t-PA-Derivats nach einem der Ansprüche 1 bis 6, **dadurch gekennzeichnet,** daß man die t-PA-DNA in einen Vektor einbringt, den erhaltenen Vektor dann denaturiert und mit einem Oligonukleotid, welches mit den Basensequenzen, die zu beiden Seiten an die aus dem t-PA-DNA-Molekül zu entfernenden Basensequenzen anschließen, komplementäre Basensequenzen aufweist, hybridisiert und einer Reparatur-Mutagenese unterwirft, den so erhaltenen mutierten Vektor in eine geeignete Zelle einführt und amplifiziert, diejenigen Vektoren gewinnt, in welchen die Deletion stattgefunden hat und den Vektor in einer geeigneten Wirtszelle exprimiert.

9. Plasmid pSV (Δ K2) -dhfr, DSM 4721

10. Plasmid pSV (ΔK2 + EGF) -dhfr, DSM 4720

11 . Mittel zur Gerinnselauflösung, enthaltend ein Gewebs-Plasminogen-Aktivator (t-PA)-Derivat, das eine Aminosäuresequenz aufweist, die der des natürlichen t-PA entspricht, in der jedoch mindestens die Aminosäuren der Domäne des Kringels II fehlen.

12 . Mittel nach Anspruch 11, **dadurch gekennzeichnet,** daß es ein t-PA-Derivat enthält, bei dem die Aminosäuren 176 bis 261 fehlen.

13 . Mittel nach Anspruch 11, **dadurch gekennzeichnet,** daß es ein t-PA-Derivat enthält, dem zusätzlich die Aminosäuren der EGF-Domäne fehlen.

14 . Mittel nach Anspruch 13 , **dadurch gekennzeichnet,** daß es ein t-PA-Derivat enthält, dem die Aminosäuren 50 bis 87 und 176 bis 261 fehlen.

# FIG.1

Humaner Plasminogen-Aktivator
vom Gewebe-Typ

|—| |———————| |————————| |——— ——— ——|     |————————————————|
Fibrin-  EGF-       Kringel I     Kringel II              Leichte Kette
finger   Domäne *   Domäne        Domäne                  (Serin-Protease)

\* Domäne mit Homologie zu
  EGF (epidermaler Wachstums-Faktor)

FIG. 2

# FIG.3

FIG. 4